(19) Europäisches Patentamt
European Patent Office
Office européen des brevets

(11) Publication number: **0 159 004**
**B1**

(12) **EUROPEAN PATENT SPECIFICATION**

(45) Date of publication of patent specification: **21.03.90**

(21) Application number: **85104546.8**

(22) Date of filing: **15.04.85**

(51) Int. Cl.$^5$: **C 07 K 5/06,** C 12 P 21/02, A 61 K 37/02, C 12 N 1/20 // (C12P21/02, C12R1:625)

(54) **Antibiotics do-248-A and B and process for preparing the same.**

(30) Priority: **16.04.84 JP 77372/84**

(43) Date of publication of application:
**23.10.85 Bulletin 85/43**

(45) Publication of the grant of the patent:
**21.03.90 Bulletin 90/12**

(84) Designated Contracting States:
**CH DE FR IT LI**

(56) References cited:
**CHEMICAL ABSTRACTS, vol. 94, no. 9, 2nd March 1981, page 767, abstract no. 66024h, Columbus, Ohio, US; T. TAKITA et al.: "Chemistry of pheganomycins, new peptide antibiotics", & PEPT. CHEM. 1977 (Pub. 1978), 15th, 121-6**

**THE JOURNAL OF ANTIBIOTICS, vol. 28, no. 10, 1975, pages 819-820, Japan Antibiotics Research Association, Tokyo, JP; R. OIWA et al.: "A new peptide antibiotic KM-8"**

(73) Proprietor: **SHIONOGI SEIYAKU KABUSHIKI KAISHA**
**12, Dosho-machi, 3-chome Higashi-ku, Osaka-shi**
**Osaka 541 (JP)**

(72) Inventor: **Kondo, Eiji**
**4-22-18, Ishibashi**
**Ikeda-cho Osaka (JP)**
Inventor: **Kawamura, Yoshimi**
**1971-9, Aomadani**
**Mino-shi Osaka (JP)**
Inventor: **Konishi, Takao**
**10-11, Tateishi-cho**
**Ikeda-shi Osaka (JP)**
Inventor: **Matsumoto, Koichi**
**6-11-72-401, Higashitoyonaka-cho**
**Toyonaka-shi Osaka (JP)**
Inventor: **Shoji, Junichi**
**1-17-14, Nagaodai**
**Hirakata-shi Osaka (JP)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 159 004 B1**

(56) References cited:
THE JOURNAL OF ANTIBIOTICS, vol. 31, May 1978, pages 410-420, Tokyo, JP; M. INUKAI et al.: "Globomycin, a new peptide antibiotic with spheroplast-forming activity"

## Description

This invention relates to the novel antibiotics DO-248-A and DO-248-B of the following Formula I:

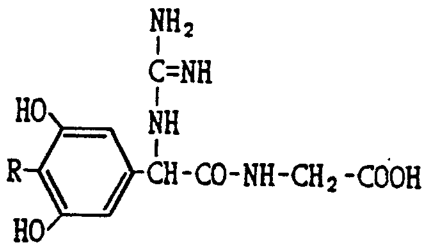

(I)

wherein R is an ethyl or isopropyl group, and their pharmaceutically acceptable salts.

Further it relates to a process for preparing these antibiotics and to pharmaceutical compositions containing as an active ingredient these antibiotics and/or their pharmaceutically acceptable salts with bases or acids. Typical examples of salts are the sodium, potassium and calcium salt, the hydrochlorides, sulfates and phosphates.

Antibiotics have long been clinically used in the chemotherapy of tuberculosis. In general, aminoglycosides such as streptomycin and kanamycin and semisynthetic macrolides such as rifampicin are used in combination and often together with tuberculostatic agents such as isoniazid.

One of the more important problems in the chemotherapy of tuberculosis is bacterial resistance. For this reason concurrent administration of two or more drugs should be employed in the treatment of all active tuberculosis diseases.

Antibiotic pheganomycins are known as antibiotics having a α-guanidino-3,5-dihydroxyphenyl acetic acid residue {Proceeding of the 15th Symposium on Peptide Chemistry, 121 (1977)}. DO-248-A and DO-248-B, however, are glycin derivatives and have a distinctly different chemical structure from pheganomycins which are hexa- or heptapeptide derivatives.

Antibiotics DO-248-A and DO-248-B of this invention have antibacterial activity against acid-resistant bacteria and kanamycin- and rifampicin-resistant bacteria and are more active than kanamycin against a typic acid-resistant pathogenic bacteria.

DO-248-A, DO-248-B, and their pharmaceutically acceptable salts are suitable for oral or parenteral administration to humans and animals. Antibiotics DO-248-A and DO-248-B are prepared by cultivating an antibiotic DO-248-A and/or DO-428-B-producing microorganism belonging to the genus Streptoverticillium in a nutrient medium and recovering antibiotic DO-248-A and/or DO-248-B from the culture broth.

Figure 1, Figure 2, Figure 3 and Figure 4 show the UV absorption spectrum in an aqueous solution, the IR absorption spectrum in a potassium bromide tablet, the $^{1}$H-NMR spectrum and the $^{13}$C-NMR spectrum of DO-248-A, respectively, and Figure 5, Figure 6, Figure 7 and Figure 8 show the UV absorption spectrum in an aqueous solution, the IR absorption spectrum in a potassium bromide tablet, the $^{1}$H-NMR spectrum and the $^{13}$C-NMR spectrum of DO-248-B, respectively.

The physicochemical properties of the antibiotics DO-248-A and DO-248-B of this invention are as follows.

(1) DO-248-A

① Elementary analysis

| | | | | |
|---|---|---|---|---|
| Calcd. (%); for $C_{14}H_{20}N_4O_5 \cdot 2H_2O$, | | C 46.66, | H 6.71, | N 15.58 |
| Found (%); | | C 47.01, | H 6.15, | N 15.44. |

② Molecular weight (by secondary ion mass spectrometry)

$(M+1)^+$ 325

③ Melting point

194—200°C

④ Specific rotation

$[\alpha]_D^{22.5}$ +84.3 ± 1.3 (C 0.966, water)

⑤ UV (see Figure 1)

$\lambda_{max}^{H_2O}$ nm ($E_{1cm}^{1\%}$) 276 (36)

$\lambda_{max}^{H_2O}$ + 1NHCl 1drop$_{nm}$ ($E_{1cm}^{1\%}$) 283 (36)

$\lambda_{max}^{H_2O}$ + 1NNaOH 1drop$_{nm}$ ($E_{1cm}^{1\%}$) 297 (74)

⑥ IR (see Figure 2)

$\nu_{max}^{KBr}$ cm$^{-1}$ 3350, 1660, 1605, 1433, 1390, 1305, 1280, 1130, 1080, 1015, 832, 803.

⑦ Solubility

soluble in water, readily soluble in methanol, ethanol and dimethylformamide, slightly soluble in chloroform, ethylacetate and acetone, insoluble in benzene, ether and hexane.

⑧ Color reaction

Sakaguchi's reaction; positive

⑨ Appearance and distinction between base, acid and neutral
colorless powder, amphoteric

⑩ $^1$H-NMR [200 MHz·$D_2O$, external reference TMS (δ value from DSS; 0.65)] (see Figure 3)
$\delta_{ppm}$ (J=Hz) 1.23 (d 6H J=7), 3.35 (m, 1H J=7), 3.61 (AB d 1H J=17), 3.83 (AB d 1H J=17), 5.10 (s 1H), 6.48 (s 2H).

⑪ $^{13}$C-NMR (δ value 67.4) (see Figure 4)
$\delta_{ppm}$ 20.5 (q × 2), 25.0 (d), 44.4 (t), 59.3 (d), 108.1 (d × 2), 123.8 (s), 134.8 (s), 156.4 (s × 2), 157.3 (s), 171.0 (s), 177.0 (s).

⑫ Amino acid analysis
Glycine and ammonia were detected.

(2) DO-248-B

① Elementary analysis

| | | | |
|---|---|---|---|
| Calcd. (%); for $C_{13}H_{18}N_4O_5 \cdot 2H_2O$, | C 45.08, | H 6.40, | N 16.18 |
| Found (%); | C 45.26, | H 6.13, | N 16.13 |

② Molecular weight (by secondary ion mass spectrometry)
$(M+1)^+$ 311

③ Melting point
189—193°C

④ UV (see Figure 5)
$\lambda_{max}^{H_2O}$ + 1NHCl 1drop$_{nm}$ ($E_{1\,cm}^{1\%}$) 276 (36), 281.5 (36)
$\lambda_{max}^{H_2O}$ + 1NNaOH 1drop$_{nm}$ ($E_{1\,cm}^{1\%}$) 297 (72)
$\lambda_{max}^{H_2O}$ + 1NNaOH 1drop$_{nm}$ ($E_{1\,cm}^{2\%}$) 297 (72)

⑤ IR (see Figure 6)
$\nu_{max}^{KBr}$ cm$^{-1}$ 3350, 1660, 1608, 1435, 1393, 1310, 1250, 1105, 1000.

⑥ Color reaction
Sakaguchi's reaction; positive

⑦ Appearance and distinction between base, acid and neutral
colorless powder, amphoteric

⑧ $^1$H-NMR [200 MHz·$D_2O$, external reference TMS (δ value from DSS; 0.65)] (see Figure 7)
$\delta_{ppm}$ (J=Hz) 1.00 (t 3H J=7.3), 2.52 (q 2H J=7.3), 3.61 (AB d 1H J=17), 3.83 (AB d 1H J=17), 5.10 (s 1H), 6.50 (s 2H).

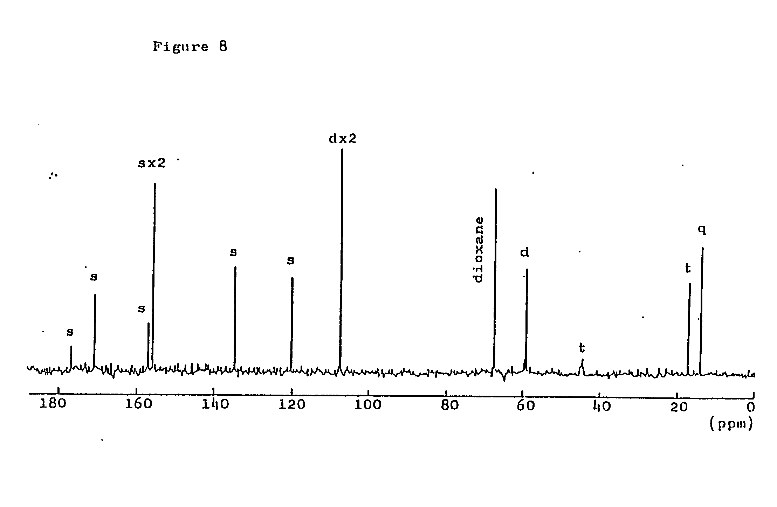

⑨ $^{13}$C-NMR [25.2 MHz, $D_2O$, internal reference dioxane (δ value 67.4)]
$\delta_{ppm}$ (J=Hz) 13.6 (q), 16.9 (t), 44.5 (t), 59.4 (d), 107.4 (d × 2), 120.1 (s), 134.8 (s), 156.0 (s × 2), 157.3 (s), 171.0 (s), 177.0 (s).
(see Figure 8).

In view of the above physiochemical properties DO-248-A and DO-248-B have the following Formula I

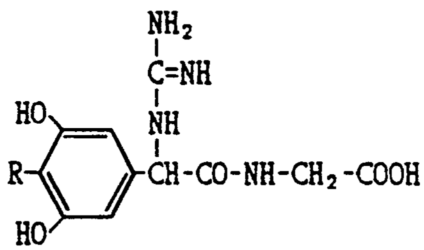

(I)

wherein R is an ethyl or isopropyl group.

DO-248-A is N-(α-guanidino-3,5-dihydroxy-4-isopropylphenylacetyl)glycine (R is isopropyl) and DO-248-B is N-(α-guanidino-3,5-dihydroxy-4-ethylphenylacetyl)glycine (R is ethyl).

DO-248-A and DO-248-B are produced by a strain belonging to Streptoverticillium isolated from a soil sample.

The microorganism belongs to the same species as Streptoverticillium roseoverticillatum (shinobu) Forina and Locci.

The taxonomical properties of the microorganism are as follows:

(1) Morphological properties (incubated in yeast extract-malt extract agar medium at 28°C for 14 days).

This microorganism grows well and forms abundant aerial mycelia, to which conidia adhere. Under the microscope branching of the aerial mycelia into a whorl is observed. The forms of the spore chains are straight and the number of conidia per chain is mostly below 10. Under an electron microscope the surface of the conidium is smooth. Any sporangia, flagellated spores and sclerotia are not observed.

(2) Cultural characteristics

Table 1

| Medium | Growth | Aerial mycelium | | Substrate mycelium | Soluble pigment |
|---|---|---|---|---|---|
| | | formation | color | | |
| Sucrose nitrate agar medium | Good | Good | Pink | None to pink | None |
| Glucose asparagine agar medium | Good | Good | Pink to pale pink | Light red | None |
| Glycerol asparagine agar medium | Good | Good | Pink | Pale red | None |
| Inorganic salt starch agar medium | Good | Good | Pinkish white to pale pink | Dull red | None |
| Tyrosine agar medium | Good | Good | Pale pink to pink | Dull red | Brownish black (trace) |
| Nutrition agar medium | Good | None | --- | Grayish brown | Grayish brown |
| Yeast extract-malt extract agar medium | Good | Good | Pale pink to pale brown | Reddish orange | Yellowish brown |
| Oatmeal agar medium | Good | Good | Pale pink to pale brown | Light red | None |
| Bennet's agar medium | Good | Good | Pale pink to pale orange | Dark reddish orange | Yellowish brown (trace) |

Colors are from GUIDE TO COLOUR STANDARD (Japan Color Institute).

(3) Physiological properties

| | |
|---|---|
| Gelatin liquefaction | negative |
| Melanin production | positive |
| Tyrosinase reaction | weakly positive |
| Coagulation of milk | negative |
| Peptonization of milk | weakly positive |
| Starch hydrolysis | positive |

(4) Utilization of carbohydrates

| carbohydrate | Growth |
|---|---|
| L-arabinose | + |
| D-xylose | + |
| D-glucose | ++ |
| D-fructose | ++ |
| Sucrose | + |
| Inositol | ++ |
| L-rhamnose | + |
| Raffinose | + |
| D-mannitol | ++ |
| Control (without sugar) | + |

++: Good growth, +: Fair growth

The microorganism is confirmed to grow fairly in the control (without sugar).

(5) Growth temperature

The microorganism grows at 14—45°C and the optimum growth temperature is 26°C to 32°C.

(6) Composition of the cell wall

Diaminopimelic acid of the LL-type was detected.

This microorganism evidently belongs to the genus Streptoverticillium deduced from several properties as mentioned above.

The closest species to the microorganism have been searched in the following references:

(1) Waxman S. A.: The Actinomycetes, vol. 2 (1961), (reference 1)

(2) Elwood B. Shirling and David Gottlieb: International Journal of Systematic Bacteriology (reference 2), vol. 18, 69—189, 279—392 (1968), vol. 19, 391—512 (1969), vol. 22, 265—394 (1972)

(3) Bergey's Manual of Determinative Bacteriology, eighth edition (1974), (reference 3)

(4) Other references referring to new species of actinomycetes.

As a result, the following 3 species are recognized as the closest species.

(1) Streptoverticillium hiroshimense (reference 2, vol. 18, 130—134 (1968); reference 3, 835)

(2) Streptoverticillium roseoverticillatum (reference 2, vol. 18, 168 (1968); reference 3, 834)

(3) Streptoverticillium biverticillatus (reference 2, vol. 18, 300 (1968); reference 3, 834).

The microorganism is examined and compared with the above 3 species. As the result it has been found that the microorganism is similar to the above 3 species and is most similar to Streptoverticillium roseoverticillatum because both have the same properties except for coagulation of milk. Accordingly, the strain DO-248 was identified to be a strain belonging to the species and named Streptoverticillium roseoverticillatum DO-248.

The strain was deposited with the Fermentation Research Institute Agency of the Industrial Science & Technology at Yatabe-machi, Tukuba-gun, Ibaraki-ken, Japan, as a national deposit under the accession

number FERM-7561 on March 26, 1984, and was transferred to the deposit under the Budapest Treaty on March 22, 1985. The strain is, therefore, now deposited under the accession number FERM BP-745 in the above depository.

This invention relates also to a process for preparing DO-248-A and/or DO-248-B using antibiotics DO-248-A- and/or DO-248-B-producing microorganisms belonging to the genus Streptoverticillium including the above DO-248 strain.

The process for preparing DO-248-A and/or DO-248-B may be performed according to a conventional fermentation process for preparing antibiotics. DO-248-A- and/or DO-248-B-producing microorganisms are incubated in a conventional medium containing several nutrients. Cultivation is carried out under aerobic conditions.

Generally, the medium contains carbon sources, nitrogen sources and inorganic salts. Optionally, e.g. vitamines and precursors can be added. Examples of carbon sources are glucose, sucrose, starch, dextrin, glycerol, molasses and organic acids. They may be employed alone or as a mixture. Examples of nitrogen sources are soy bean meal, corn steep liquor, meat extract, yeast extract, cottonseed powder, peptone, wheat germ, ammonium sulfate and ammonium nitrate. They may be employed alone or as a mixture. Optionally, inorganic acids such as calcium carbonate, sodium chloride, potassium chloride, magnesium sulfate, cobalt chloride, several phosphates can be added to the medium.

The cultivation may be performed according to conventional methods. In this invention a liquid culture is particularly preferred. For mass production submerged aerated culture is preferred. The pH of the medium is in the range of about 5.5 to about 8.5, the temperature of the fermentation is in the range of 20°C to about 40°C, preferably about 25°C to about 32°C. The cultivating time greatly depends on the scale of the fermentation and is preferably about 20—80 hours in a large scale production.

If necessary, an antifoaming agent such as vegetable oil and the like may be added before or during the fermentation.

After termination of the cultivation, DO-248-A and DO-248-B can be isolated and recovered from the culture medium according to conventional methods, e.g. by filtration, centrifugation, adsorption and desorption and chromatography using active adsorbents. Suitable solvents for the extraction are organic solvents and their mixtures. The salts are prepared in a conventional manner by reacting the antibiotics with a base or an acid.

DO-248-A and B provided by this invention show potency activity against acid-resistant bacteria, in particular pathogenic mycobacteria including tubercle bacilli. The minimum inhibitory concentration of DO-248-A for various pathogens is as follows.

Method of the test

Acid-resistant bacilli (0.01 mg) shown in Table 1 were inoculated into 2.0 ml of Dubos Tween albumin liquid medium and the minimal inhibitory concentration (MIC, μg/ml) of DO-248-A (Compound A), kanamycin (KM) and rifampicin (RFP) was measured by the two-fold dilution method. The incubation temperature for Mycobacterium marinum was 28°C and that for all other microorganisms was 37°C. The evaluation was made generally 2 weeks later but 1 week later on M. furtuitum and M. chelonei and 5 weeks later on M. xenopi.

Table 2

| Test Microorganism | | MIC (μg/ml) | | |
|---|---|---|---|---|
| Species | Strain | Compound A | K M | R F P |
| M. tuberculosis | H37Rv | 0.05 | 0.025 | <0.001 |
| 〃 | R-15[1] | 0.10 | <0.001 | >25 |
| 〃 | R-18[2] | 0.025 | >25 | >25 |
| M. bovis | BCG | 0.05 | 0.00156 | <0.001 |
| M. kansasii | KMC1101 | 0.00313 | 6.25 | 0.0125 |
| M. marinum | KMC1202 | 12.5 | 3.13 | 1.56 |
| M. scrofulaceum | KMC2102 | 0.2 | 0.0125 | 0.00313 |
| M. gordonae | KMC2201 | 0.00625 | 0.39 | 0.0125 |
| M. szulgai | KMC2401 | 3.13 | 1.56 | 0.025 |
| M. xenopi | KMC2301 | 0.39 | 0.10 | 0.00625 |
| M. simiae | KMC1302 | 6.25 | 12.5 | >25 |
| M. avium | KMC3101 | 0.78 | 3.13 | 0.20 |
| M. intracellulare | KMC3209 | 0.025 | 0.05 | <0.001 |
| M. nonchromogenicum | KMC3602 | 12.5 | >25 | 25 |
| M. fortuitum | KMC4101 | >25 | 25 | 3.13 |
| M. chelonei | KMC4201 | >25 | >25 | 6.25 |
| M. avium-intracellulare | clinical isolated strain16 | 0.10 | 6.25 | 3.13 |

1) Rifampicin resistant strain

2) Kanamycin and rifampicin resistant strain

As shown in Table 2, the antibiotic DO-248-A of this invention is effective against strains resistant to kanamycin and rifampicin and more effective than kanamycin and rifampicin against M. avium-intracellulare, which may cause a disease with symptoms that resemble chronic bronchitis. DO-248-B is as effective as DO-248-A. Accordingly, these antibiotics and their pharmaceutically acceptable salts can be used as pharmaceuticals for the treatment of infections caused by acid-resistant bacteria.

DO-248-A, DO-248-B, and their pharmaceutically acceptable salts are administered orally or parenterally to humans or animals in the form of e.g. tablets, capsules, powders or liquids formulated with

common pharmaceutically acceptable excipients, stabilizers, preservatives, wetting agents, surfactants, flavoring agents, fragrances and e.g. as injection preparations or suppositories. The dosage varies depending on the severity of the disease, sex, age, weight, etc. About 0.2 to about 8 g/day are preferable for a normal adult. The compounds of this invention may be administered together with other antibacterial agents.

Examples for preparing the compounds of this invention, DO-248-A and DO-248-b, are given below. However, they should not be understood to restrict this invention.

Example 1

a) Fermentation step

S medium: 0.5% soluble starch, 0.5% glucose, 0.5% polypeptone, 0.5% meat extract, 0.25% yeast extract, 0.25% sodium chloride, deionized water (pH 7.0 before sterilization).

X medium: 2.0% raw starch, 2.0% glucose, 2.0% defatted soy bean powder, 0.5% yeast extract, 0.25% sodium chloride, 0.35% calcium carbonate, 0.0005% manganese dichloride tetrahydrate, 0.0005% cupper sulfate pentahydrate, 0.0005% zinc sulfate heptahydrate.

One platinum loop of Streptoverticillium roseoverticillatum DO-248 strain (FERM BP-745) was inoculated into a 500 ml Sakaguchi's flask in which 100 ml of the above S medium was charged and cultured at 28°C for 48 hours with shaking. Each 4 ml portions of this medium was inoculated into 500 ml Sakaguchi's flasks charged with 100 ml of the above X medium and cultured at 28°C for 4 days with shaking to give 43.8 L of the cultured medium.

a) Isolation step

To 43.8 L of the cultured medium provided in the above step was added 1.2 kg of a filter aid, Hyflo Super Cel® (Johns-Manville Sales Corp.) and the cells were filtered off. The filtrate (39 L) was passed through a column with 4.36 L of a synthetic adsorbent HP-20 (150—300 μm, Mitsubishi Chemical Industries Co., Ltd.) to absorb the desired ingredient. The column was washed with water and eluted with methanol to give active fractions, which were concentrated under reduced pressure. The active fractions in the first half contaminated with many water soluble impurities were subjected to column chromatography with 580 ml of a synthetic adsorbent CHP-20P (75—150 μm, Mitsubishi Chemical Industries Co., Ltd.). The resulting active fractions were combined and lyophilized to yield 25 g of a brown crude powder. Then, 25 g of this crude powder is adsorbed to a column with 470 g of silicagel (63—200 μm, Merck & Co.) and eluted with chloroform:methanol:water (15:10:1) to remove impurities and then with methanol to elute the active ingredient. The active fractions were evaporated to dryness in vacuo. 3.18 g of the resulting yellowish brown residue containing the active ingredient was dissolved in a small amount of water and passed through a column of 700 ml of Sephadex LH-20 (Pharmacia AB). The column was washed with water and eluted with methanol. The eluate was evaporated to dryness in vacuo. The resulting powder (1.22 g) is dissolved again in a small amount of water and passed through a column with Toyopearl HW-50C (100—500 μm, Toyo Soda Mfg. Co., Ltd.) to absorb the active ingredient. The column was washed with water and then eluted with methanol. The active fractions were evaporated to dryness in vacuo (according to this purification impurities are not removed completely, but DO-248-B, the minor component, was collected in a fair amount from the first half fractions). Each crude powders were combined and passed through reverse phase Lobar® column (Lichroprep R-18®, 40—63 μm, 25 × 310 mm, Merck & Co.) with methanol-water (1 l) to remove impurities and resulting active fractions were evaporated to dryness in vacuo to provide 445 mg of yellow powder. At last the resultant powder was separated into DO-248-A and DO-248-B by reverse phase high performance liquid chromatography with a semi-collection column. Nucleosil® $10C_8$ (10 × 250 mm, M. Nargel Co., Ltd.), (it takes 14—15 minutes and 7—8 minutes to elute DO-248-A and DO-248-B, respectively, with methanol-water (1:2) at a flow rate of 3 ml/minute. A UV 254 nm detecter was employed). The eluates were then lyophilized to yield 116 mg and 16 mg of colorless powder of DO-248-A and DO-248-B, respectively.

Example 2

a) Fermentation step

S medium: 0.5% soluble starch, 0.5% glucose, 0.5% polypeptone, 0.5% meat extract, 0.25 yeast extract, 0.25% sodium chloride, deionized water (pH 7.0 before sterilization).

A medium: 2% raw starch, 2% glucose, 2% defatted soy bean powder, 1% Bactosoyton (Trade name), 0.25% sodium chloride, water (pH 7.0 before sterilization).

A 2 L Erlenmeyer flask charged with 800 ml of S medium consisting of the above composition was inoculated with a seed culture of Streptoverticillium roseoverticillatum DO-248 (FERM BP-745) and incubated at 28°C for 24 hours under shaking (180 r.p.m.).

Into 30 L jars charged with 20 L of A medium having the above composition, 800 ml portions of this culture medium were added and incubated with 20 L/minute aeration and 180—300 r.p.m. at 28°C for 5 days.

b) Isolation step

The culture medium provided in the above step was adjusted to pH 3.0 and centrifuged with a Sharples

type centrifuge to give 100 L of a supernatant. The supernatant was passed through a column of 10 L of Dowex® 50 × 4 ($NH_4^+$ type) (U.S., Dow Chemical Co.) at a flow rate of 700 ml/minute. After washing with water, the column was eluted with 0.3 N aqueous ammonia to give 35 L of active fractions. After ammonia was removed, the fractions were adjusted to pH 9.0 and passed through a column of 5 L of Dowex 1 × 2 ($Cl^-$ type) at a flow rate of 150 ml/minute. The column was eluted with 50% methanol containing 5% sodium chloride. 13 L of the resulting active fractions were evaporated in vacuo. The residual solution was adjusted to pH 7.0 and passed through a column of 2.5 L of HP-20 (Mitsubishi Chemical Industries Co., Ltd.). After washing with water, the column was eluted with 50% methanol and 2.7 L of the active fractions were evaporated. The resultant concentrate was lyophilized to yield 2.25 g of crude powder of DO-248.

c) Purification step of DO-248-A

Detection and quantitation of DO-248-A in the following purification step were performed with high performance liquid chromatography tracing the absorbance at 230 nm, employing a column (4 × 250 nm) of Nucleosil® $10C_8$ (M. Nargel Co., Ltd.) and a mixture of 20 mM phosphate buffer (pH 7.0) and acetonitrile (5 l). Under the experimental conditions the retention volume of DO-248-A was 8.00 ml.

The crude powder (2.0 g) provided in the above step (containing about 105 mg of DO-248-A) was dissolved in about 40 ml of 20 mM phosphate buffer (pH 7.0). After removing a small amount of impurities, this solution was poured on a column (200 ml) of QAE-Sephadex A-25 (Pharmacia AB). After 200 ml of 20 mM phosphate buffer (pH 7.0) passed through the column, a linear concentration gradient elution was performed with 600 ml of the same buffer and 600 ml of the same containing 1M sodium chloride. The eluting solution was traced with the above high performance liquid chromatography to give fractions containing DO-248-A. The collected eluates were applied to a column (100 ml) of CHP-20P (Mitsubishi Chemical Industries Co., Ltd.). The column was washed with water and then a linear concentration gradient elution was performed with 300 ml of water and 300 ml of 80% methanol. The eluting solution was traced with high performance liquid chromatography to give fractions containing DO-248-A. The collected eluates were evaporated in vacuo and then lyophilized to yield 100 mg of a powder whose purity was about 70%. About 50 mg portions of this powder was subjected to a high performacne liquid chromatography collection column (Nucleosil® $30C_{18}$, 20 × 250 mm) at one time and developed with a mixture of 50 mM phosphate buffer (pH 7.0) and methanol (9:1). The fractions were checked by their absorbance at 230 nm and those fractions containing the desired substance in a peak were collected. This collected solution was passed through a column (40 ml) of CHP-20P and the column was washed with water and eluted with 50% methanol. The eluates containing DO-248-A were collected by detecting with the above high performance liquid chromatography, evaporated in vacuo and then lyophilized to give 60 mg of a colorless powder of DO-248-A.

**Claims**

1. A compound of Formula I:

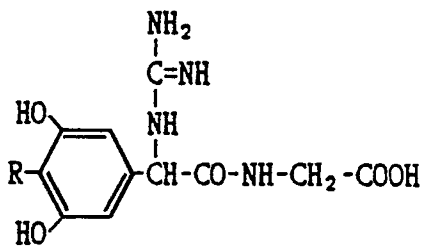

(I)

wherein R is ethyl or isopropyl, and the pharmaceutically acceptable salt thereof.

2. The compound of Claim 1 wherein R is isopropyl, antibiotic DO-248-A.

3. The compound of Claim 1 wherein R is ethyl, antibiotic DO-248-B.

4. A process for preparing antibiotic DO-248-A and/or DO-248-B which comprises incubating an antibiotic DO-248-A- and/or DO-248-B-producing microorganism belonging to the genus Streptoverticillium in a nutrient medium and recovering antibiotic DO-248-A and/or DO-248-B from the culture medium.

5. The process of Claim 4 wherein the medium is a liquid medium.

6. The process of Claim 4 wherein the medium is adjusted to about pH 5.5 to about pH 8.5.

7. The process of Claim 4 wherein the cultivation is performed at about 20°C to about 40°C.

8. The process of Claim 4 wherein the cultivation is performed at about 25°C to about 32°C.

9. The process of Claim 4 wherein the cultivation is performed for about 20 to about 80 hours.

10. The process of Claim 4 wherein a microorganism belonging to the genus Streptoverticillium and having the identifying characteristics of the strain FERM BP-745 is used.

11. Microorganism Streptoverticillium roseoverticillatum DO-248 (FERM BP-745).

12. Pharmaceutical composition containing as an active ingredient antibiotic DO-248-A and/or DO-248-B, or a pharmaceutically acceptable salt thereof.

**Patentansprüche**

1. Verbindung der allgemeinen Formel I

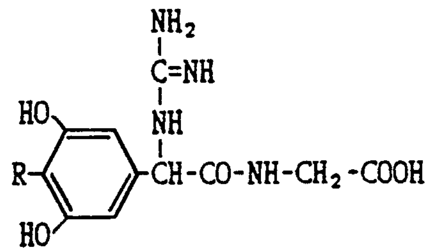

(I)

in der R eine Äthyl- oder Isopropylgruppe ist und das pharmazeutisch verträgliche Salz davon.

2. Verbindung nach Anspruch 1, in der R eine Isopropylgruppe ist, nämlich das Antibiotikum DO-248-A.

3. Verbindung nach Anspruch 1, in der R eine Äthylgruppe ist, nämlich das Antibiotikum DO-248-B.

4. Verfahren zur Herstellung der Antibiotikum DO-248-A und/oder DO-248-B, dadurch gekennzeichnet, daß man einen das Antibiotikum DO-248-A und/oder DO-248-B bildenden Mikroorganismus der Gattung Streptoverticillium in einem Nährmedium züchtet und das Antibiotikum DO-248-A und/oder DO-248-B aus dem Kulturmedium wiedergewinnt.

5. Verfahren nach Anspruch 4, in dem das Medium ein flüssiges Medium ist.

6. Verfahren nach Anspruch 4, in dem das Medium auf einen pH-Wert von etwa 5,5 bis etwa 8,5 eingestellt ist.

7. Verfahren nach Anspruch 4, in dem die Züchtung bei etwa 20°C bis etwa 40°C durchgeführt wird.

8. Verfahren nach Anspruch 4, in dem die Züchtung bei etwa 25°C bis etwa 32°C durchgeführt wird.

9. Verfahren nach Anspruch 4, in dem die Züchtung etwa 20 bis etwa 80 Stunden durchgeführt wird.

10. Verfahren nach Anspruch 4, in dem ein Mikroorganismus der Gattung Streptoverticillium mit den identifizierenden Merkmalen des Stammes FERM BP-745 verwendet wird.

11. Mikroorganismus Streptoverticillium roseoverticillatum DO-248 (FERM BP-745).

12. Arzneimittel, enthaltend als Wirkstoff das Antibiotikum DO-248-A und/oder DO-248-B oder ein pharmazeutisch verträgliches Salz davon.

**Revendications**

1. Composé de formule I:

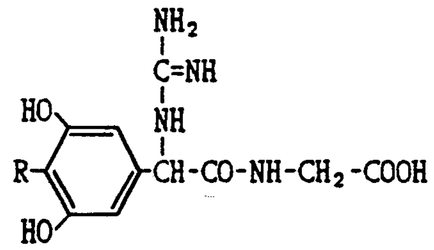

(I)

dans lequel R est un groupe éthyle ou isopropyle, et le sel acceptable pharmaceutiquement de celui-ci.

2. Composé selon la revendication 1, caractérisé en ce que R est un groupe isopropyle, à savoir l'antibiotique DO-248-A.

3. Composé selon la revendication 1, caractérisé en ce que R est un groupe éthyle, à savoir l'antibiotique DO-248-B.

4. Procédé pour la préparation de l'antibiotique DO-248-A et/ou pour DO-248-B comprenant l'incubation dans un milieu nutritif, d'un microorganisme produisant l'antibiotique DO-248-A et/ou DO-248-B, appartenant à l'espèce Streptoverticillium, et la récupération de l'antibiotique DO-248-A et/ou DO-248-B à partir du milieu de culture.

5. Procédé selon la revendication 4 caractérisé en ce que le milieu est un milieu liquide.

6. Procédé selon la revendication 4 caractérisé en ce que le milieu est ajusté à un pH d'environ 5,5 à un pH d'environ 8,5.

7. Procédé selon la revendication 4 caractérisé en ce que la culture est réalisée d'environ 20°C à environ 40°C.

8. Procédé selon la revendication 4 caractérisé en ce que la culture est réalisée d'environ 25°C à environ 35°C.

9. Procédé selon la revendication 4 caractérisé en ce que la culture est realisée pendant environ 20 à environ 80 heures.

10. Procédé selon la revendication 4 caractérisé en ce qu'on utilise un microorganisme appartenant à l'espèce Streptoverticillium et possédant les caractéristiques d'identification de la souche FERM BP-745.

11. Microorganisme Streptoverticillium roseoverticillatum DO-248 (FERM BP-745).

12. Composition pharmaceutique contenant en tant qu'ingrédient actif l'antibiotique DO-248-A- et/ou DO-248-B, ou un sel pharmaceutiquement acceptable de celui-ci.

Figure 1

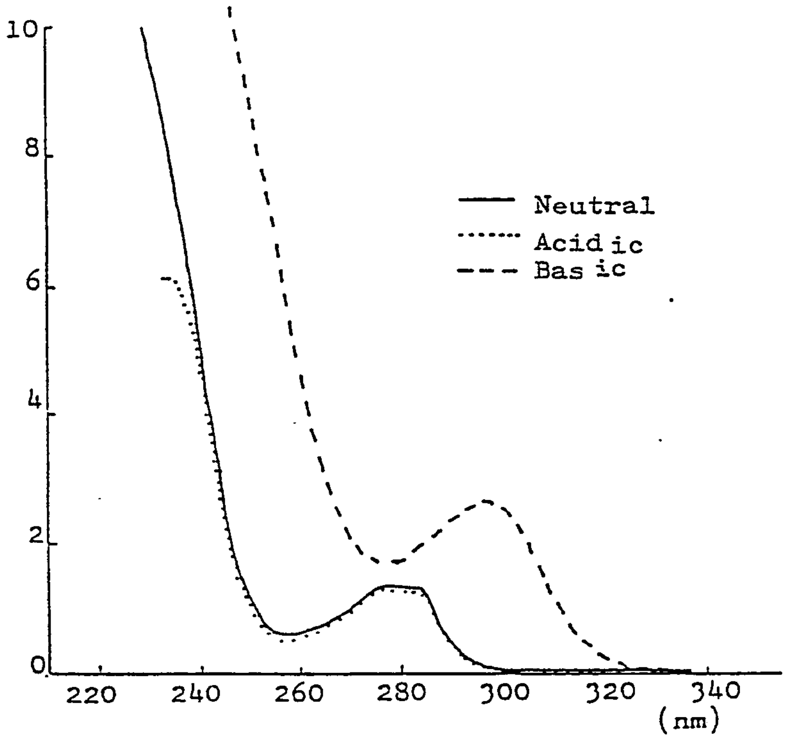


Figure 2

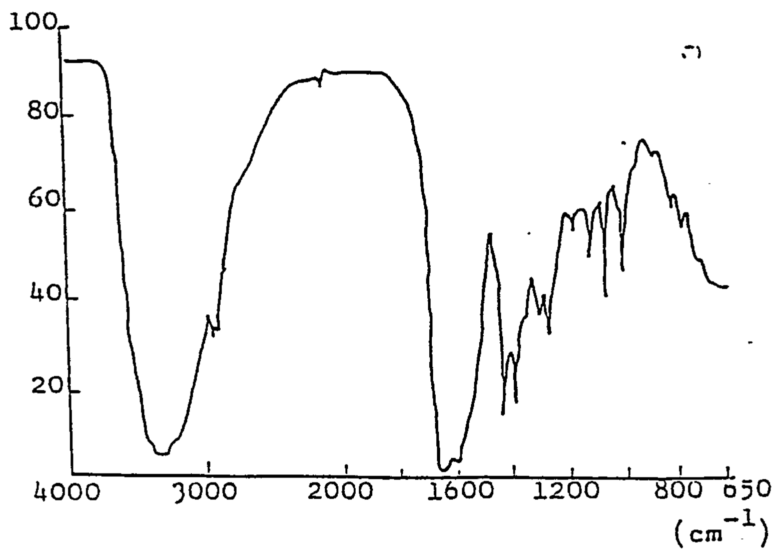

Figure 3

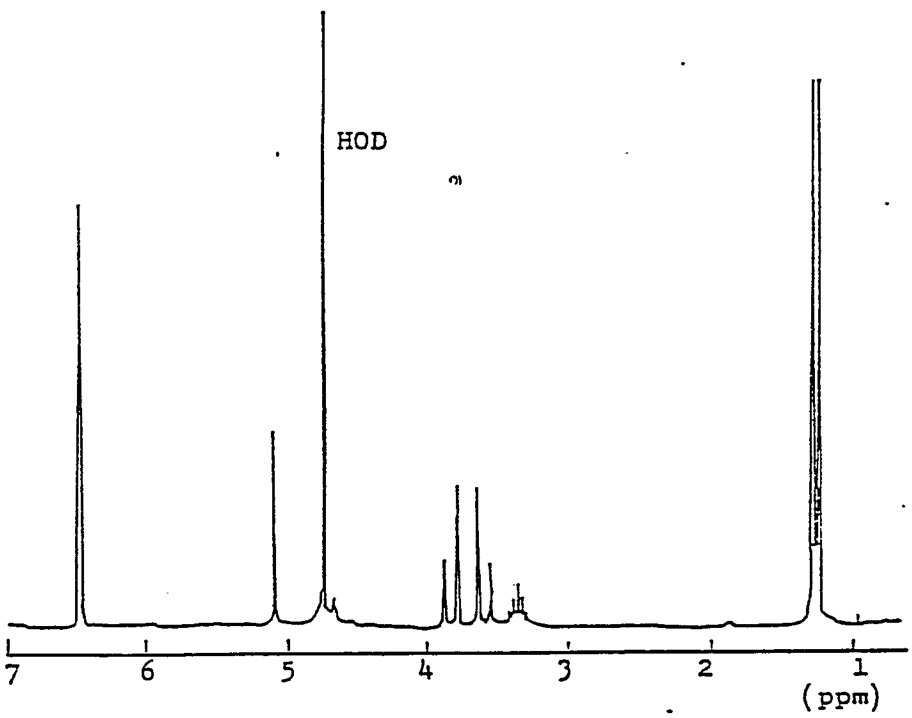

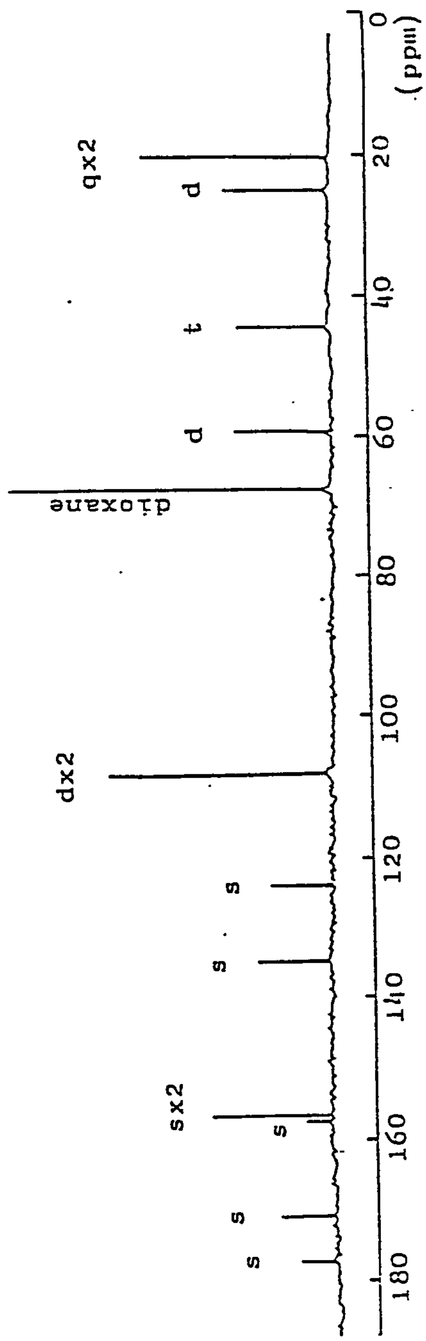
qx2
d
t
d
dioxane
dx2
s
s
sx2
s
s
s
0
20
40
60
80
100
120
140
160
180
(ppm)

Figure 4

Figure 5

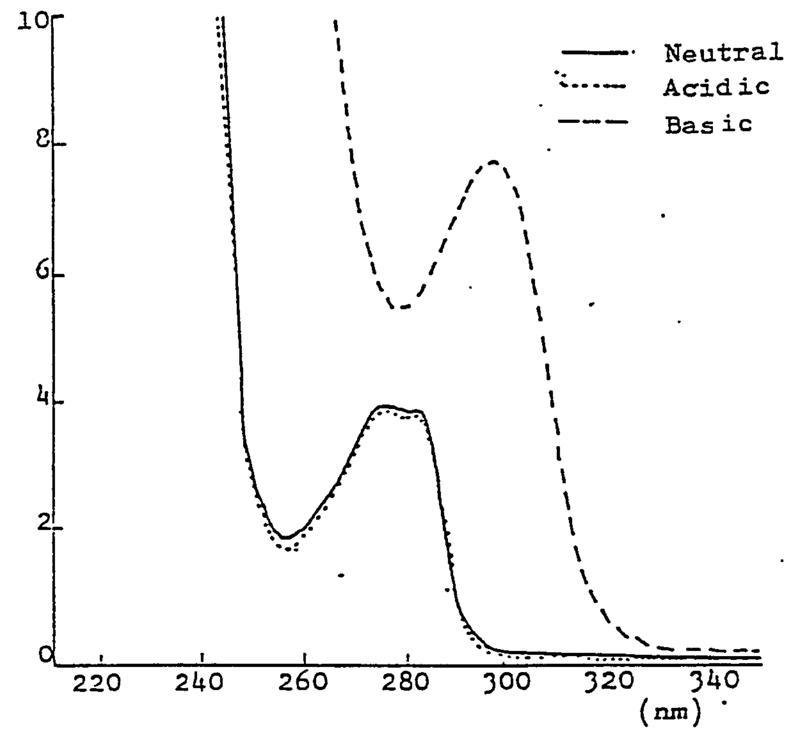


Figure 6

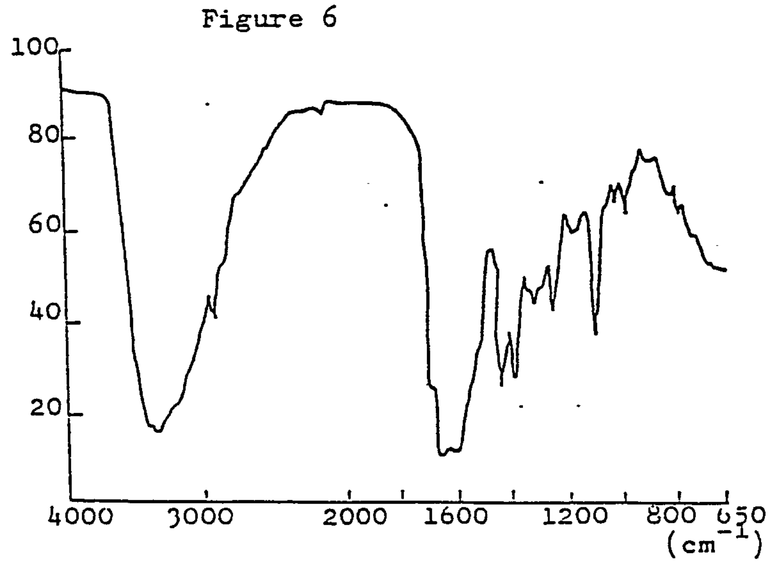

Figure 7

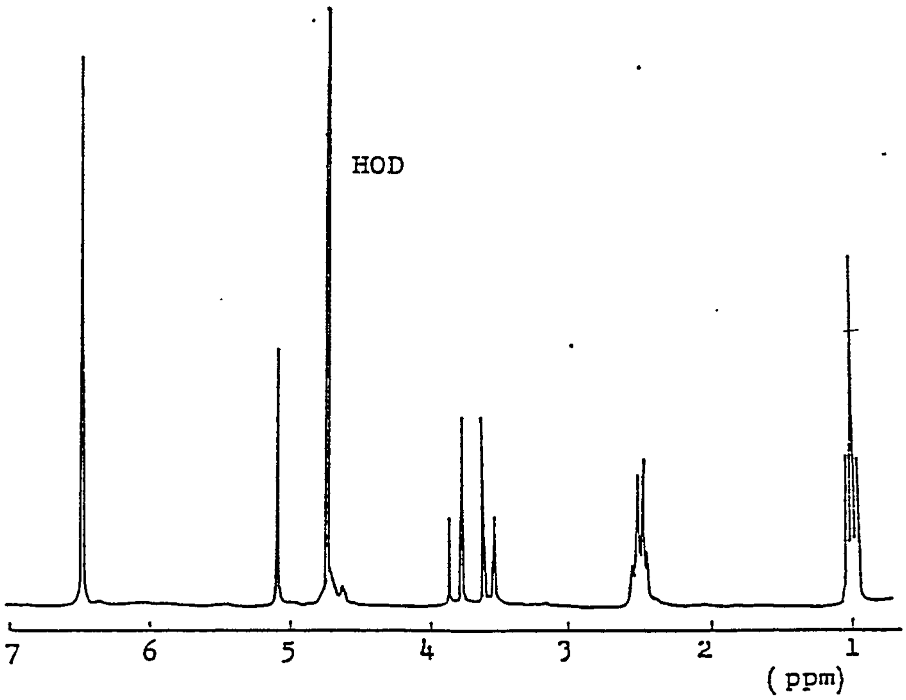

Figure 8